# EUROPÄISCHE PATENTSCHRIFT

(11) **EP 0 594 655 B1**
(45) Veröffentlichungstag und Bekanntmachung des Hinweises auf die Patenterteilung: **06.03.1996**
(21) Anmeldenummer: 92913780.0
(22) Anmeldetag: 03.07.1992
(51) Int. Cl.: A61N 2/04

(54) **VORRICHTUNG ZUM TRANSPORT VON IONEN, INSBESONDERE PROTONEN**
DEVICE FOR TRANSPORTING IONS, ESPECIALLY PROTONS
DISPOSITIF POUR LE TRANSPORT D'IONS, EN PARTICULIER DE PROTONS

(30) Priorität: 09.07.1991 DE 4122718
(43) Veröffentlichungstag der Anmeldung: 04.05.1994
(73) Patentinhaber: DR. FISCHER AG, FL-9490 Vaduz (LI)
(72) Erfinder: WARNKE, Ulrich, D-6601 Scheidt (DE); FISCHER, Gerhard, FL-9490 Vaduz (LI); KÖNIG, Herbert, L., D-8000 München 40 (DE)
(74) Vertreter: Engelhardt, Guido, Dipl.-Ing.
(86) Internationale Anmeldenummer: DE9200564
(87) Internationale Veröffentlichungsnummer: WO9300960

(56) Entgegenhaltungen:
- EP-A- 0 136 530
- EP-A- 0 377 284
- DE-A- 2 707 574
- DE-A- 3 244 582
- DE-A- 3 335 018
- US-A- 3 566 877
- US-A- 4 654 574
- US-A- 4 818 697
- ELEKTROTECHNIK Bd. 64, Nr. 4, Februar 1982, WUERZBURG DE Seite 11 'Einfluss magnetischer Felder auf den Organismus'

## Beschreibung

Die Erfindung betrifft eine Vorrichtung mit einem niederfrequent gepulste elektrische Ströme erzeugenden Generator und einer daran angeschlossen Sendespule, deren elektromagnetischen Felder zur Beaufschlagung einer zu behandelnden Körperregion dienen.

Vorrichtungen dieser Art sind aus der Literatur und auch aus Patentschriften allgemein bekannt. So beschreibt die US-A-1 4 428 366 einen elektromagnetischen Apparat und eine Methode zur Herabsetzung des Glukosepegels im Blutserum mit unipolaren gepulsten Magnetfeldern niedriger Pulsfolgefrequenz, die zwischen 5 und 75 Hz liegt, bei einer Dauer des Einzelimpulses von z.B. 350 Mikrosekunden. Der einzelne Impuls hat dabei eine Impulsdachschräge, um ein konstantes Magnetfeld für die Dauer des Einzelpulses sicherzustellen. Dies wird mit einer Anzeige überwacht, die von einer im Bestrahlungsbereich vorgesehenen Meßspule gespeist wird.

In der US-PS 4 641 633 A1 ist ein elektronisches System zur Aktivierung, Beeinflussung und/oder Veränderung und der Entwicklung von Zellen, Organen und des Organismus lebender Individuen behandelt. Nach dieser Druckschrift finden hierzu unipolare Rechteckimpulse hoher Flankensteilheit mit einer Pulsfolgefrequenz zwischen 1 und 120 Hz Anwendung, die über eine Antenne in den zu behandelnden Bereich eingestrahlt werden. Den Einzelpulsen ist dabei eine Tastung mit einer hochfrequenten Schwingung überlagert, deren Frequenz zwischen 10 kHz und 100 MHz liegen soll.

In der EP-O 152 963 A2 ist ein Elektrotherapiegerät beschrieben, bei dem an die zu behandelnde Körperregion Elektroden angelegt werden, denen niederfrequent gepulste elektrische Schwingungen zur Erzeugung von elektromagnetischen Feldern dem Körpergewebe zugeführt werden. Das Gerät arbeitet im wesentlichen nach dem Gewebeerwärmungsprinzip in Kombination mit einem elektromagnetischen Wechselfeld.

In der DE-PS 1 38 28 043 A1 ist ein medizinisches Behandlungsgerät zur Beeinflussung magnetoenergetischer Vorgänge im lebenden menschlichen oder tierischen Organismus beschrieben, bei dem in einem flexiblen flachen Kissen eine oder mehrere Induktionsspulen untergebracht sind, die mit pulsierendem Gleichstrom betrieben werden. Der pulsierende Gleichstrom erzeugt ein niederfrequent pulsierendes Magnetfeld, dessen Frequenz z.B. bei 15 bis 20 Hz liegt.

In der EP-O 266 907 A2 ist ein Apparat zur Bestrahlung eines Patientenkörpers behandelt, bei dem eine hochfrequente Schwingung von z.B. 27 MHz niederfrequent getastet wird. Die Tastfrequenz soll zwischen 1 Hz und 10 kHz liegen, bei einer Einzelimpulsdauer zwischen etwa 10 und 100 Mikrosekunden.

In der CH-PS 675 970 A5 ist schließlich ein Gerät zur Behandlung von Lebewesen mit einem intermittierenden und pulsierenden magnetischen Gleichfeld beschrieben. Es werden Pulspakete aus mehreren Einzelimpulsen abgestrahlt und die Impulse können im jeweiligen Pulspaket in der Amplitude konstant, ansteigend, abfallend oder an- und abschwellend verlaufen.

Allen diesen Vorrichtung und Verfahren ist gemeinsam, daß sie auf die Beeinflussung von biologischen Vorgängen mit Erwärmung und/oder elektromagnetischer Strahlung gerichtet sind.

Es ist ferner bekannt, daß an den Blutgefäßen im Koronar- und Halsbereich sogenannte Baro-Rezeptoren sitzen, mit deren Hilfe der Körper den Blutdruck steuert, und daß es möglich ist, diese Barorezeptoren mit Hilfe von elektromagnetischen Feldern so zu beeinflussen, daß der Blutkreislauf aktiviert wird. Es konnte auf diese Weise auch eine Erweiterung der Kapillaren erzielt werden, was zu einer verbesserten Durchblutung der entsprechenden Körperbereiche führte. Da die Barorezeptoren nur an bestimmten Stellen der Blutgefäße sitzen, sind aber deren Einflußmöglichkeiten recht begrenzt. Eine verbesserte Ver- und Entsorgung bestimmter Gefäße und Körperbereiche kommt damit nur indirekt über eine allgemein verbesserte Durchblutung zustande. Eine Beeinflussung des Lymphgefäßsystems ist nicht möglich, da dieses nicht über Baro-Rezeptoren verfügt.

Demgegenüber liegt der Erfindung die Aufgabe zugrunde, eine Vorrichtung zum Transport von Ionen und insbesondere von Protonen anzugeben, mit der eine gezielte Beeinflussung der Ionenkonzentration in beliebigen Körperbereichen bei Menschen und Tieren ermöglicht wird.

Diese Aufgabe wird, ausgehend von einer Vorrichtung der einleitend beschriebenen Art, erfindungsgemäß dadurch gelöst, daß die Sendeenergie so hoch gewählt ist, daß die in der Elektrolythflüssigkeit induzierte Energie höher ist als die thermische Energie und innerhalb der Grenzwerte des sogenannten zellspezifischen Amplitudenfensters liegt, und daß die vom Generator in der, vorzugsweise induktionsarm ausgebildeten Sendespule erzeugten gepulsten Ströme folgende Eigenschaften aufweisen:
- der Grundstrompuls besteht aus der Überlagerung eines Rechteck-Stromes und eines etwa nach einer e-Funktion ansteigenden Stromes, gefolgt von einer wenigstens gleich langen Pulspause,
- die Grundfrequenz der Grundstromimpulse mit Grundpulspausen beträgt 100 bis 1000 Hz, vorzugsweise 200 Hz,
- die Amplitude der Grundpulsfolge ist mit einer Modulationsfrequenz von 0,5 bis 25 Hz, vorzugsweise 20 Hz in der Amplitude moduliert,
- die modulierte Grundpulsfolge wird als Pulsfolgeserie für eine Zeitdauer von 0,3 bis 1,0 sec. ausgesendet, woran sich jeweils eine pulsserienpause von 0,7 bis 5,0 sec. anschließt.

Die bekanntesten Vertreter der intrakorporalen Elektrolyt-Flüssigkeiten bei Menschen und Tieren sind das Blut und die Lymphe. Das Blut ist im Körper das universelle Transportmittel für Sauerstoff, Kohlendioxid, Wasser, Salze und andere Elektrolyte, Nährstoffe, Stoffwechselprodukte, Wärme, katalytisch wirkende Stoffe wie Hormone und Enzyme, Antikörper, Wundverschlußstoffe usw.. Seinem Fließverhalten nach ist es eine Nicht-Newtonsche Flüssigkeit, eher vergleichbar einer Emulsion als einer Suspension. Der pH-Wert beträgt etwa 7,38. Die relative Dielektrizitätskonstante liegt entsprechend dem hohen Wassergehalt bei 80, jedenfalls im niederfrequenten Bereich. Die Lymphe ist eine farblose bis gelbliche Körperflüssigkeit, die aus dem Blutplasma entsteht und durch die Kapillaren in das Gewebe abgegeben wird. Sie umgibt alle Zellen. Sie sammelt sich in Gewebespalten und Hohlräumen. Die Ableitung erfolgt zunächst in kleinen Lymphkapillaren, die sich zu größeren Lymphgefäßen zusammmenschließen. Bevor diese wieder in den Blutkreislauf einmünden, passieren sie die Lymphknoten. Die Lymphe gibt Nährstoffe an das Gewebe und transportiert Stoffwechselprodukte aus dem Gewebe ab. Die Lymphe enthält ca. 95 % Wasser. Als weitere wichtige intrakorporale Flüssigkeit wäre zu nennen der Liquor, der Hirn und Rückenmark umspielt.

Von besonderer Bedeutung für die einwandfreie Funktion eines menschlichen und tierischen Organismus ist das sogenannte Säure-Basen-Gleichgewicht, das innerhalb enger Grenzen konstant gehalten werden muß, da sonst erhebliche Funktionsstörungen, z. B. des Ionen-Antagonismus, der Sauerstoff-Transportfunktion des Blutes, der Zellmembran-Durchlässigkeit im Gewebe, der Eigenschaften der Enzyme usw., eintreten können. Das Säure-Basen-Gleichgewicht wird beschrieben durch die sogenannte Henderson-Hasselbach'sche Gleichung. Das Säure-Basen-Gleichgewicht steht im engem Zusammenhang mit dem allgemeinen Elektrolythaushalt des Körpers.

Dank der kombinierten Wirkung von Impulsfrequenz, Impulsform, Impulsenergie und Sendespulenform ist es möglich, Ionen, insbesondere Protonen, aus der intrakorporalen Elektrolyt-Flüssigkeit, beispielsweise dem Blut, der Lymphe oder dem Liquor, gezielt und unmittelbar in die sie umgebenden Gefäßwände und Membranen einzuschleusen. Dies ist unter normalen Bedingungen nicht möglich, da die Lipide in den Membranen der Blutgefäße, die in Kontakt mit dem Blut stehen, eine negative Ladung tragen. Das dadurch ausgelöste Feld übersteigt die thermische Energie und reicht etwa 1000 A weit in das Plasma. Die Konzentration von Kationen nahe an der Gefäßoberfläche ist etwa eine Zehnerpotenz höher als in der Plasmaphase. Dadurch ist der lokale pH-Wert um eine Zehnerpotenz niedriger als in der Elektrolyt-Flüssigkeit.

Normalerweise stellt das Oberflächenpotential eine elektrische Barriere dar, die Protonen und andere Ionen hindert, in die Gefäßwände einzudringen. Die Energie, die ein Ion mit einem Radius von 10⁻¹⁰ m benötigt, um aus Wasser in ein Lipidmedium überzutreten, beträgt ca. 22,6 eV. Das Oberflächenpotential der Gefäßwände und Membranen im Körper variiert sehr stark. Dem wird durch die Amplitudenmodulation der Grundpulsfolgen Rechnung getragen. In diesem Zusammenhang ist auch zu beachten der sogenannte Fenstereffekt, d. h. Daß die induzierten Spannungen nur dann das Eindringen von Ionen und Protonen in die Gefäßwand ermöglichen, wenn ihre Stärke innerhalb eines zellspezifischen Amplitudenfensters liegt. Zu kleine aber auch zu große Amplituden verhindern einen Ionentransport durch die Gefäßwände und Membranen.

Die Existenz zellspezifischer Amplitudenfenster ist seit langem bekannt, so beispielsweise durch eine Veröffentlichung von Adey in der Zeitschrift "Proceedings of the IEEE", 68,1,119-125 aus dem Jahre 1980 und durch eine Veröffentlichung von Basset in der Zeitschrift "Ortopädie" 13, 64-77 aus dem Jahr 1984.

Die _{"}zellspezifischen Amplitudenfenster" sind außerdem durch folgende Literaturstellen allgemein bekannt:
Bawin S.M., Adey W.R., 1976 _{"}Sensitivity of calcium binding in cerebral tissue to weak environmetal electromaganetic fields oscillating at low frequency" Proceed.Nat.Acad.Sci. USA 73: 1999.2003
Bawin S.M., Sheppard A.R., Adey W.R. 1978, _{"}Possible mechanism of weak elektromaganetic field coupling in brain tissue" Bioelektrochem. Bioenergetics 5: 67-76
Blackman C.S. et al 1979, _{"}Induction of calcium Ion efflux from brain tissue by radiofrequency radiation, effects of modulation frequency and field strength" Radio Sci. 14: 93:98
Ferner ist das viele Einzelarbeiten zusammenfassende Buch von Adrew A. Marino, _{"}Modern Bioelectricity", erschienen 1988 bei Marcel Dekker Inc., New York und Basel, insbesondere Seiten 181-193 und 357-356, zu nennen. In diesem Buch wird sehr umfangreich auf die verschiedenen Transportmechanismen und deren energetische Betrachtung eingegangen.

Unter dem Einfluß der erfindungsgemäßen, pulsierenden elektromagnetischen Felder wird in der Elektrolyt-Flüssigkeit eine elektrische Spannung bestimmter Stärke und Richtung induziert. Unter deren Einfluß bewegen sich die Ionen und wegen ihrer höheren Beweglichkeit bevorzugt die Protonen in Richtung auf die Gefäßwände. Aufgrund der dadurch entstehenden Wechselwirkung wird das induzierte Feld auf eine kleine Fläche konzentriert. Dadurch entsteht eine sogenannte Konzentrationspolarisation.

Durch die Wahl der Exponential-Funktion (e-Funktion) für die Amplitude der einzelnen Grundpulse ergibt sich die überraschende und für die medizinischbiologische Wirkung äußerst bedeutsame Konsequenz, daß die Spannungsimpulse induziert werden, deren Form im wesentlichen gleich ist und die vor allem keine Phasenverschiebung gegenüber den Strom- bzw. Feldpulsen besitzen. Lediglich am Ende jedes Grundstromimpulses wird ein sehr kurzer Spannungsimpuls von umgekehrter Polarität induziert, der jedoch den positiven Effekt nur geringfügig stört. Dank der Tatsache, daß die Sendestrompulse bzw. das von ihnen erzeugte Magnetfeld und die induzierten Spannungspulse gleiche Form und gleiche Phase besitzen, wird ein Maximum an Energie übertragen. Zusätzlich ergibt sich der überraschende Effekt, daß sowohl die positiven als auch die negativen Ionen der Elektrolyt-Flüssigkeit sich in die gleiche Richtung bewegen. Üblicherweise sind die Bewegungsrichtungen von positiven und negativen Ionen einander entgegengesetzt. Nach dem erfindungsgemäßen Verfahren können also positive und negative Ionen aus der Elektrolyt-Flüssigkeit des Körpers gleichzeitig in dieselben Zellen transportiert werden. Die in der Gefäßwand induzierte Spannung wird durch das Polarisationspotential verstärkt. Aufgrund der Tatsache, daß die Gefäßwände und insbesondere deren Membranen sehr dünn sind, entstehen sehr hohe Feldstärken, auch wenn die induzierten Spannungen absolut gesehen relativ klein bleiben. So führt beispielsweise eine induzierte Spannung von nur 30 mV in einer Membran von 200 nm Dicke zu einer Feldstärke von 150 kV/m. Es sei darauf hingewiesen, daß Feldstärken dieser Größenordnung nur auf induktivem Wege mit elektromagnetischen Feldern, auf keinen Fall auf kapazitiven oder galvanischem Wege mit Elektroden erzielt werden können. Aufgrund der hohen Leitfähigkeit der interkorporalen Elektrolyt-Flüssigkeiten bilden die Gefäße gleichsam einen Faradayschen Käfig, dessen Inneres frei von elektrischen Feldern bleibt. Die induktive Erregung dagegen bedient sich gerade der elektrischen Leitfähigkeit der intrakorporalen Elektrolyt-Flüssigkeiten zur Erzeugung der Spannungen und Felder.

Die schon erwähnte negative Ladung der Gefäßwände gegenüber den Elektrolyt-Flüssigkeiten ist auch bedingt durch die Unterschiede der relativen Dielektrizitätskonstanten der Gefäßwände einerseits und der Flüssigkeiten andererseits. Blut und Lymphe haben aufgrund ihres hohen Wassergehalts relative Dielektrizitätskonstanten in der Größenordnung von 80. Die Gefäßwände besitzen relative Dielektrizitätskonstanten in der Größenordnung von 3 bis 5. Die erfindungsgemäß in den Elektrolyt-Flüssigkeiten induzierten Spannungen und Ströme bzw. Felder sind in der Lage, diese Zeta-Potential genannte Potentialschwelle zu neutralisieren. Dadurch können nun Ionen und insbesondere die beweglichen Protonen verstärkt in die Zell- und Gefäßwände übertreten. Durch die Anreicherung der Protonen in den Zell- und Gefäßwänden kommt es dort zur Ausbildung einer umgekehrt polarisierten Potentialschwelle, die die Protonen und Ionen daran hindert, die Zell- und Gefäßwände wieder zu verlassen.

Durch die veränderte Protonen-Konzentration kommt es außerdem zu einer günstigen Veränderung des pH-Wertes, speziell im Bereich der Gefäßwände.

Alle diese Effekte sind besonders wirksam, je dünner die Gefäßwände sind. Sie sind also besonders stark im Bereich der arteriellen Kapillaren, wo bekanntlich der Austausch des vom Blut mitgeführten Sauerstoffs gegen das von den Zellen abgegebene Kohlendioxid stattfindet.

Neben den bisher beschriebenen Effekten mit Langzeitwirkung haben die elektromagnetischen Felder weitere Wirkungen. Es seien hier nur genannt die Elektrostriktion der Membranen und Gefäßwände infolge Körperschall mit Ansteuerung von Mechano- und Piezo-Rezeptoren, das Ausrichten von polyvalenten Ionenketten, die tangentiale Verschiebung von adsorbierten Gegen-Ionen, die Kraftwirkung auf dielektrische Körper in homogenen und inhomogenen Feldern sowie die Elektroosmose.

Die Grundfrequenz der Grundstrompulse ist vorzugsweise abgestimmt auf die mechanische Resonanz der Blut- und Lymphgefäße.

Als optimale induzierte Amplitudenform haben sich gleichschenklige Dreiecke bewährt, wobei gemäß einer Weiterbildung die Polarität der Amplitude nicht wechselt. Um derartige Impulse induzieren zu können, bedarf es spezieller Stromkurven aber auch spezieller Sendespulen. Vorteilhafterweise werden den Grundpulsen Hochfrequenzpulse einer Frequenz von ca. 10 - 100 kHz überlagert. Diese Frequenz ist abgestimmt auf die kapazitive Überleitung durch die Membranen hindurch.

Um die durch die induzierten Spannungen und Felder ausgelösten Effekte optimal wirken zu lassen, benötigt der Organism bestimmte Pausen. Vorteilhaft werden deshalb die Grundpulse in regelmäßigen Folgen ein- und ausgeschaltet, wobei die Ein- und Auszeiten zwischen 0,3 sec.: 0,7 sec. Und 0,7 sec. : 5,0 sec. Variiert werden können.

Durch eine Anpassung der Feld-Parameter kann die Wirkung auf den Organismus optimiert werden. Vorteilhafterweise wird mit Biofeedback geregelt.

Zu diesem Zweck wird gemäß einer ersten Varianten ein Blutdruckmeßgerät an die erfindungsgemäße Vorrichtung angeschlossen. In diesem Fall erfolgt die Regelung auf einen optimalen Wert des Blutdrucks.

Gemäß einer zweiten Varianten wird ein Thermograph angeschlossen. In diesem Fall erfolgt die Regelung auf eine optimale Erwärmung des gewünschten Körperbereichs durch verbesserte Durchblutung.

Gemäß einer dritten Varianten wird ein Pulsmeßgerät angeschlossen. Damit wird die Erkenntnis ausgewertet, daß bei optimaler Wirkung der gepulsten elektromagnetischen Felder sich der Pulsschlag verlangsamt.

Gemäß einer vierten Varianten wird ein AtemvolumenMeßgerät angeschlossen. Damit wird die Erkenntnis ausgewertet, daß bei einer Absenkung des pH-Wertes im Liquor sich das Atemvolumen erhöht, d. h. sich die Atmung vertieft.

Auf externe Hilfsvorrichtungen kann jedoch verzichtet werden, wenn erfindungsgemäß die induktionsarm aufgebaute, als biegsame Flachspule ausgebildete Sendespule mit wenigstens einer induktiven Meßspule kombiniert wird. Diese muß ebenfalls äußerst induktionsarm sein, um die im Organismus induzierten äußerst schwachen Felder möglichst unverzerrt aufnehmen zu können.

Diese Meßwicklung nimmt das vom Organismus mehr oder weniger stark reflektierte und phasenverschobene Magnetfeld auf. Das Meßsignal wird mit einer geeigneten hochempfindlichen elektronischen Auswerteschaltung ausgewertet, worauf mit Hilfe eines gegebenenfalls eingebauten Reglers die Parameter der Sendestromimpulse optimiert werden.

Nicht nur die Form der Sendespule, sondern auch die Form der Meßspule ist von Bedeutung für eine optimale Funktion und Wirkung der elektromagnetischen Felder im Organismus. Überraschenderweise hat sich nun herausgestellt, daß die Wirkungen im Organismus dann optimal sind, wenn die Sendestrompulse so stark gewählt werden, daß in einer kreisförmigen Meßspule mit nur einer Windung und einem Durchmesser von 20 cm eine Meßspannung von 20 bis 30 mV induziert werden. Dabei muß allerdings vorausgesetzt werden, daß der von den elektromagnetischen Feldern beeinflußte Bereich des Organismus ebenfalls eine Ausdehnung von ca. 20 cm oder mehr besitzt. Sollen kleinere Körperbereiche gemessen werden, beispielsweise Arme oder Beine, muß eine entsprechend kleinere Meßspule eingesetzt werden. Die dadurch systembedingt geringere Meßspannung wird in die Regelschaltung eingeeicht.

Bezüglich Form und Konstruktion der Sendespule hat sich überraschenderweise herausgestellt, daß dann, wenn die Windungen der Sendespule eine ovale Spirale bilden und auf beiden Seiten der Trägerplatte verteilt sind, sich die besten Ergebnisse erzielen lassen.

Dank einer extrem induktionsarmen Konstruktion ist die Sendespule in der Lage, die optimalen Pulsformen, -frequenzen und -leistungen verzerrungsfrei abzustrahlen.

In allen Fällen sollte die Form der Sendespule derart sein, daß sich im bestrahlten Organismus an den Wirkungsstellen die erforderlichen Spannungen und Felder aufbauen können, ohne daß es zu gefährlichen örtlichen Feldspitzen kommt. In diesem Sinne ist die Ausgestaltung der Sendespule als sogenannter Quadrupol optimal.

Demselben Zweck dient auch eine Anpassung der Trägerplatte, die die Windungen der Sendespule trägt, an das zu behandelnde Körperteil.

Anhand der Zeichnung wird die Erfindung in Form von Ausführungsbeispielen näher erläutert. Es zeigen, jeweils in schematischer Darstellung
- Fig. 1: eine Draufsicht auf eine erste Sendespule,
- Fig. 2: eine Draufsicht auf eine Sendespule als Quadrupol,
- Fig. 3: eine Draufsicht auf eine Vielfachspule,
- Fig. 4: eine Draufsicht auf eine Vielfach-Meßspule,
- Fig. 5: eine Ausführung einer Sendespule für die Behandlung von großflächigen Körperpartien,
- Fig. 6: eine Ausführung einer Sendespule für die Behandlung von Armen und Beinen,
- Fig. 7: den Aufbau dieser Sendespule als Sprengbild,
- Fig. 8: einen optimalen Grundstrompuls mit zugehöriger Grundpulspause,
- Fig. 9: eine induzierte Pulsform als Spannungs-Zeit-Diagramm,
- Fig. 10: die Grundpulse der Fig. 8 in anderem Zeitmaßstab und amplitudenmoduliert, und
- Fig. 11: die Pulsfolgesereie in wieder anderem Zeitmaßstab, und
- Fig. 12 bis 14: Schaltungsdetails der in Figur 5 nicht näher dergestellten Grundeinheit und von der Einstellung dienenden Schaltungen.

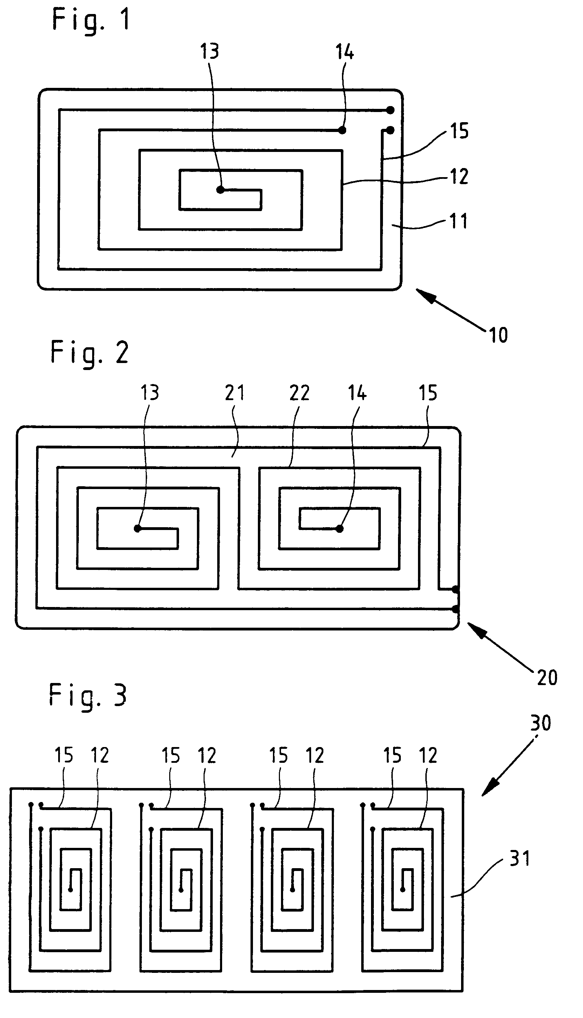

Fig. 1 zeigt eine Draufsicht auf eine erste Ausführungsform einer Sendespule 10. Auf einer Trägerplatte 11 aus hochflexiblem Isoliermaterial befindet sich die eigentliche Sendewicklung 12 in Form einer ovalen Spirale, hier der Einfachheit halber mit rechteckigem Querschnitt wiedergegeben. Das innere Wicklungsende 13 ist durchkontaktiert zu einer auf der Rückseite der Trägerplatte 11 aufgebrachten weiteren Wicklung mit dem selben Wicklungssinn. Die Stromzuführung erfolgt am äußeren Wicklungsende 14.

Um die Sendespule 12 herumgelegt ist eine induktive Meßwicklung 15. Diese nimmt das im zu behandelden Organismus zum Teil intensitätsgeschwächte und phasenverschobene reflektierte Feld auf und führt es zu einer geeigneten elektronischen Schaltung. Geeignete hochempfindliche Schaltungen sind bekannt, z. B. in Form der Phasen-Synchron-Anlagen.

Fig. 2 zeigt eine zweite Ausführungsform einer Sendespule 20. Auf der Trägerplatte 21 befinden sich zwei ovale Spiralen 22 mit gegenläufigem Wicklungssinn. Entsprechende Wicklungen befinden sich auf der Rückseite der Trägerplatte 21. Dank dieser speziellen Wicklungsform bildet sich ein sogenannter Quadrupol aus, dessen Feldlinien noch besser geeignet sind, die gewünschten Effekte im Organismus zu erzielen. Auch hier ist eine Meßwicklung 15 vorgesehen.

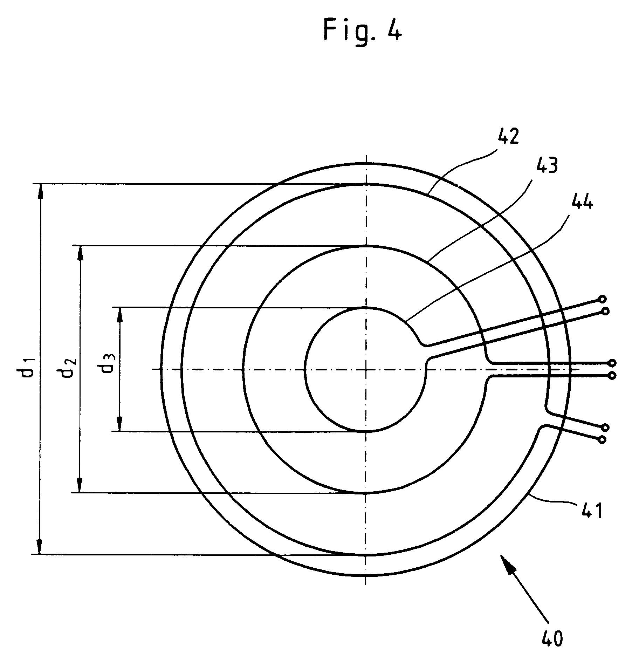

Fig. 3 zeigt eine dritte Ausführungsform einer Sendespule 30, die speziell zur Verwendung in der klinischen Praxis bestimmt ist. Auf einem wieder hochflexiblem Träger 31, dessen Größe etwa der Größe einer Bettdecke entsprechen kann, sind im vorliegenden Beispiel vier Sendespulen 12 mit zugehöriger Meßwicklung 15 angeordnet. Durch Zu-und einzelner Sendespulen können mehr oder weniger große Körperbereiche gleichzeitig behandelt werden. Ein Überzug verhindert Beschädigungen und Verschmutzungen.

Anhand der Fig. 4 soll ein interessanter und grundlegender Aspekt der vorliegenden Erfindung behandelt werden.

Dargestellt ist eine Sendespule 40 mit einem flexiblen Träger 41, wobei jedoch die Spiralwicklungen der Sendespule 40 nicht dargestellt sind. Dargestellt sind drei Meßwicklungen 42, 43, 44 mit unterschiedlichen Durchmessern d1, d2, bzw. d3. Die durchgeführten Versuche haben gezeigt, daß die Wirkung der gepulsten elektromagnetischen Felder auf den Organismus dann optimal ist, wenn in einer kreisförmigen Meßwicklung mit nur einer Windung und einem Durchmesser von 20 cm eine Spannung von 20 bis 30 mV induziert wird, vorausgesetzt, der bestrahlte Körperbereich ist entsprechend groß. Sollen großflächigere Körperbereiche behandelt werden, würde eine zu kleine Meßwicklung nur einen Teil der reflektierten Energie aufnehmen, was eine ungenügende Sendeenergie vortäuschen würde. Aus diesem Grunde werden in der Praxis Meßwicklungen mit unterschiedlichen Durchmessern eingesetzt, wobei die dadurch systembedingten unterschiedlichen Induktionsspannungen in die angeschlossene Meßapparatur eingeeicht werden.

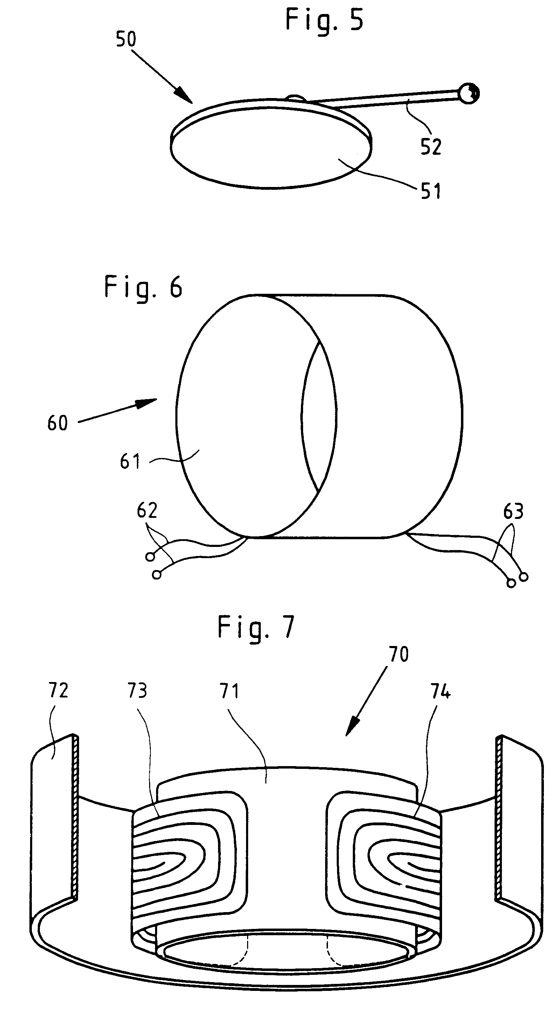

Fig. 5 zeigt eine erste Ausführung einer Sendespule 50 für die ärztliche Praxis. An einem Gelenkarm 52 ist eine körpergerecht geformte Platte 51 befestigt, in derem Innerem sich Sendespulen und die Meßwicklungen befinden. In einem (nicht dargestellten) Grundgerät finden Stromversorgung, Generator, Meßvorrichtung, Regelvorrichtung und Bedienteil ihre Plätze.

Fig. 6 zeigt eine weitere Ausführungsform einer Sendespule 60, hier speziell zur Behandlung von Armen und Beinen, beispielsweise nach Knochenbrüchen. Die Sendespule 60 ist als Zylinder 61 ausgebildet, an den Stromzuführungen 62 und Meßleitungen 63 angeschlossen sind.

Fig. 7 zeigt eine zylinderförmige Sendespule 70 als Sprengbild. Zwischen einem Innenring 71 und einem Außenring 72, beide aus isolierendem Material bestehend, befinden sich die eigentlichen Sendewicklungen 73, 74. Jede dieser Wicklungen bildet eine ovale Spirale.

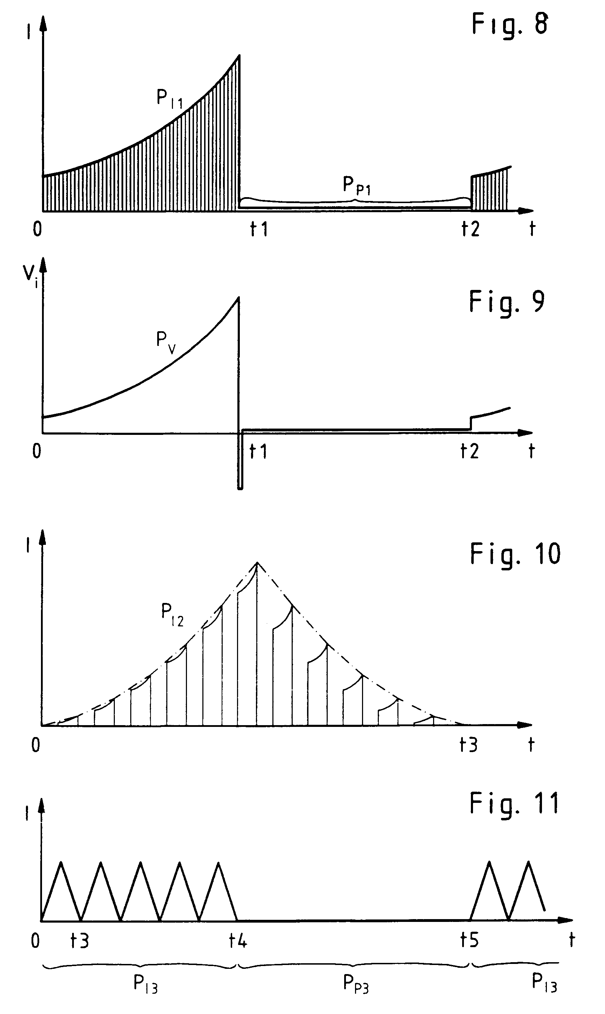

Fig. 8 zeigt als Amplituden-Zeit-Diagramm (Strom I, Zeit t) die optimale Form eines Grundstromimpulses P_{I1} gefolgt von einer Grundpulspause P_{P1}. Die Dauer eines Grundstromimpulses P_{I1} entspricht der Zeitspanne 0 bis t₁, die Dauer einer Grundpulspause entspricht der Zeitspanne t₁ bis t₂. Das Puls-Pausen-Verhältnis beträgt ca. 2:3. Die Frequenz der Grundpulse liegt zwischen 100 und 1000 Hz., Vorzugsweise bei 200 Hz.

Den Grundstrompulsen P_{I1} sind Hochfrequenzpulse mit einer Frequenz zwischen 10 und 100 kHz überlagert. Die Frequenz dieser Hochfrequenzimpulse ist abgestimmt auf die kapazitive Überleitung in den Gefäßen des Organismus. Das Wesentliche der Grundstrompulse P_{I1} jedoch ist ihre nach einer e-Funktion ansteigende Amplitude. Diese Kurvenform hat zwei bedeutungsvolle und überraschende Konsequenzen. Zum einen sind die im Organismus induzierten Spannungsimpulse P_{V} formgleich, zum anderen sind sie mit den Strompulsen in Phase.

Dies ist im Amplituden-Zeit-Diagramm der Fig. 9 dargestellt. Die Formgleichheit zwischen Grundstrompuls P_{I1} und induziertem Spannungsimpuls P_{V} wird lediglich gestört durch einen sehr kurzen Störimpuls am Ende des Grundstrompulses zum Zeitpunkt t₁. Auf der Ordinate ist hierbei als Größe die induzierte Spannung Vᵢ aufgetragen.

Dank der Phasengleichheit von Strom und Spannung wird ein Maximumm an elektrischer Energie in den Organismus übertragen. Aufgrund der physiologischen Gegebenheiten im Organismus selbst, bedingt durch die Führung des Blutes in langgestreckten Blutgefäßen, stellt sich jedoch noch ein weiterer Effekt ein, der darin besteht, daß sowohl die positiven als auch die negativen Ionen in die gleiche Richtung transportiert werden. Damit wird es erstmals möglich, die Zellen des Organismus mit beiden Komponenten einer dissozierten chemischen Substanz zu versorgen.

Fig. 10 zeigt in verkleinertem Zeitmaßstab eine komplette Grundpulsfolge P_{I2}, deren Amplitude mit einer Modulationsfrequenz von 0,5 bis 35 Hz, vorzugsweise 20 Hz moduliert ist, wobei die Modulation selbst etwa ein gleichschenkliges Dreieck ohne Polaritätswechsel darstellt.

Figur 11 zeigt in einem noch kleineren Zeitmaßstab eine komplette Pulsfolgeserie P_{I3}, deren Zeitdauer der Zeit 0 bis t₄ entspricht, gefolgt von einer Pulsserienpause P_{P3} der Zeitdauer t₄ bis t₅. Das Ein-Aus-Verhältnis kann variiert werden zwischen 0,3 : 0,7 sec. Und 0,7 : 5,0 sec. Durch die Pulsserienpause P_{P3} wird der Tatsache Rechnung getragen, daß der Organismus jeweils eine gewisse Zeit benötigt, um die von der Pulsfolgeserie P_{I3} eingeleiteten chemisch-physikalischen Vorgänge wirksam werden zu lassen.

Die Frequenz der Grundstrompulse P_{I1} ist auf die mechanische Resonanz der Blut- und Lymphgefäße abgestimmt. Die Hochfrequenz ist auf die kapazitive Überleitung durch die Gefäßwände und Membranen hindurch abgestimmt. Von wesentlicher Bedeutung ist, daß die Pulsamplitude so stark gewählt wird, daß im Organismus die nötigen Spannungen und Feldstärken induziert werden, wobei jedoch das richtige Fenster eingehalten werden muß. Dies wird mittels Meßwicklung überwacht. Dabei ist die elektrische Leitfähigkeit der bestrahlten Körperregion, die sich an einem erhöhten Reflexionsfaktor manifestiert, ein Maß für die verbesserte Durchblutung, während die Phasenverschiebung zwischen Strom und Spannung des Meßsignals ein Maß ist für die veränderte Polarisation der Gefäßwände und Membranen.

Es versteht sich, daß die physiologischen Wirkungen der erfindungsgemäßen elektromagnetischen Felder auch durch die bekannten medizinischen Meßvorrichtungen kontrolliert werden können, beispielsweise durch ein Blutdruckmeßgerät, ein Pulsmeßgerät, einen Thermografen oder auch einen Respirografen.

Wie die Versuche gezeigt haben, sind die Form und Konstruktion der Sendespulen von besonderer Bedeutung für die optimale Wirkung des erfindungsgemäßen Gerätes. Obwohl die Grundfrequenz mit vorzugsweise 200 Hz sehr niedrig ist, treten durch das Ein- und Ausschalten hochfrequente Oberwellen auf. Bei einer nicht-optimierten Spulenform werden diese formbestimmenden Oberwellen nicht ausreichend übertragen, d.h. die Impulsform wird verändert und die Wirkung verschlechtert sich. Ein induktionsarmer Aufbau der Sendespulen ist daher wichtig. Auch müssen durch die Formgebung der Sendespulen etwaige Feldlinienkonzentrationen verhindert werden, die zu einer Schädigung des Organismus Anlaß sein könnten. Da die Wirkung der Magnetfelder richtungsabhängig ist, müssen die Sendespulen richtig appliziert werden.

Wie die Versuche gezeigt haben, lassen sich grundsätzlich alle biologischen Organismen behandeln. Organismen mit ausgebildetem Blut- oder Lymphkreislauf, d. h. Menschen und Säugetiere, sind dabei bevorzugt. Mit Hilfe des erfindungsgemäßen Geräts können beispielsweise bei Reit- und Springpferden die Muskelbildung und die Gelenkregeneration, bei Kühen die Milcherzeugung und bei Schweinen die Fleischbildung angeregt werden. Bei der Behandlung von Menschen ist die Verwendung des Gerätes in der Medizin und im Sport angezeigt, insbesondere nach Knochenbrüchen.

Folgende Einflüsse der erfindungsgemäßen gepulsten elektromagnetischen Felder auf den Organismus infolge des durch sie bewirkten Transports von Ionen, insbesondere von Protonen, aus dem Blut in die angrenzenden Gewebe und Elektrolythräume, insbesondere im Zusammenhang mit dem beschriebenen Effekt, daß sowohl positive als auch negative Ionen in die gleiche Richtung wandern, konnten bereits verifiziert werden:
Durch die pH-Wert-Absenkung, ausgelöst durch die Protonenanreicherung, werden die Baro-Rezeptoren sensibilisiert, wobei sich dieser Effekt addiert zu der bereits bekannten mechanischen Erregung der Baro-Rezeptoren mittels Elektrostriktion. Dadurch wird die Erregung des Sympatikus gesenkt, die Gefäßdilatation wird verstärkt, die Wärmeabstrahlung von der Oberfläche des Organismus wird (meßbar) erhöht und der Sauerstoffpartialdruck in der behandelten Körperregion erhöht.

Gleichzeitig wird durch die pH-Wert-Absenkung das Vaguscenter sensibilisiert, wodurch die Pulsfrequenz meßbar sinkt.

Eine pH-Wert-Absenkung aktiviert auch die Makrophagen.

Wird auch der pH-Wert des Liquor gesenkt, wird das Atemzentrum sensibiliert und es kommt zu einer meßbaren Vertiefung der Atmung.

Die Wirkung der gepulsten elektromagnetischen Felder ist optimal, wenn das Blut eine hohe Protonenkonzentration besitzt. Dies ist der Fall im Schlaf, da dann das Blut viel CO₂ enthält, nach körperlicher Anstrengung oder auch nach Alkoholkonsum, da dann das Blut eine hohe Laktat-Konzentration aufweist, und beim Fasten, da dann das Blut viel Ketose enthält.

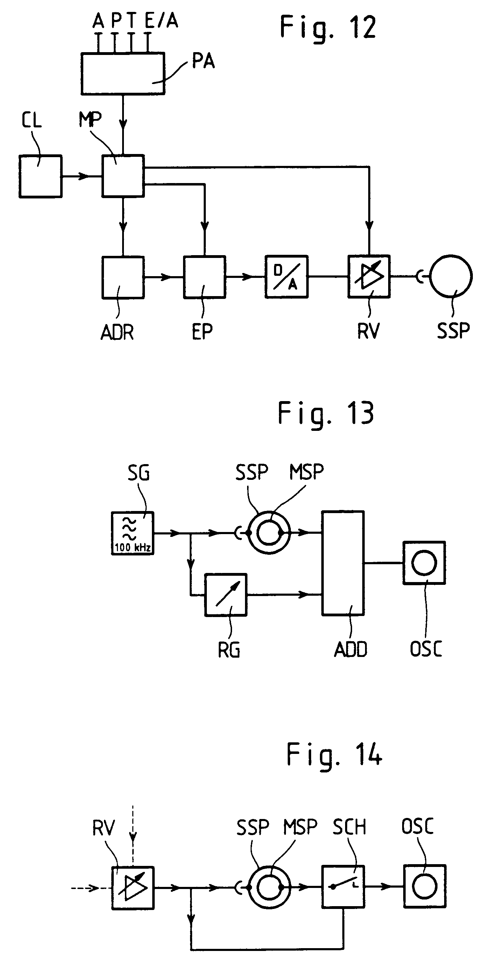

Wie bereits erwähnt, zeigen die Figuren 12 bis 14 Schaltungsdetails, des anhand der Figur 5 bereits erwähnten Grundgeräts und der ebenfalls bereits erwähnten Meß- bzw. Regeleinrichtungen.

In der Figur 12 ist ein Blockschaltbild einer erprobten Grundeinheit wiedergegeben. Als Ablaufsteuerung ist für die Grundeinheit ein Mikroprozessor MP vorgesehen, der seine Einstellgrößen von einem Bedienungspult PA aus erhält. Dem Mikroprozessor MP wird der Arbeitsakt mit einem mit C1 bezeichneten Taktsignalgenerator zugeführt. Die gewünschte Form des zu erzeugenden Sendesignals ist als Folge von einzelnen Amplitudenwerten in digitaler Form in einem mit EP bezeichneten Speicherbaustein, z.B. einem EPROM fest abgespeichert.

Vom Mikroprozessor MP wird dem Speicher EP ein Auslesesignal zugeführt, das ein Auslesen derjenigen Speicherzellen veranlaßt, deren Speicheradresse vom Mikroprozessor MP über einen Adressen-Speicher und -Generator ADR festgelegt wird. Am Ausgang des Speichers EP wird daher beim Betrieb eine Folge von digitalen Werten bzw. Wörtern abgegeben, die die gewünschte Form des Sendesignals beschreibt. Dieses digitale Signal wird mittels eines Digital-Analogwandlers A/D in eine Folge von entsprechenden Amplitudenproben umgewandelt, die über einen Tiefpaßverhalten aufweisenden Verstärker RV, der außerdem in seiner Verstärkung regelbar ist, als kontinuierliches Signal einer Sendespule SSP zugeführt wird.

Vom Bedienungspult PA aus ist über einen Einsteller A die Verstärkung des Tiefpaßverstärkers RV und damit die Amplitude des der Sendespule SSP zugeführten Stromes einstellbar. Mit dem Einsteller P ist über den Mikroprozessor MP die Folge von nacheinander auszulesenden Speicherzellen des Speichers EP festlegbar, die die gewünschte Signalform am besten beschreibt. Mit dem Einsteller Z sind die geforderten Zeitwerte der Pulse wählbar, die über den Mikroprozessor MP einzustellen sind. Mit E/A ist der Betriebsschalter bezeichnet, mit dem das Grundgerät ein- bzw. ausgeschaltet werden kann. Die Erzeugung von Signalen besonderer Form in dieser Weise ist an sich beispielsweise durch die DE-A1 3 628 219 bekannt, so daß sich eine weitergehende Beschreibung erübrigt.

In der Figur 13 ist die vorstehend schon erwähnte Meßeinrichtung zur Bestimmung der Impedanz einer zu behandelten Körperregion als Blockschaltbild wiedergegeben. Ein Signalgenerator SG mit einer Frequenz von z.B. 100 kHz speist eine Sendespule SSP, der eine Meßspule MSP zugeordnet ist. Das der Sendespule SSP zugeführte Signal und das der Meßspule MSP entnommene Signal werden gleichphasig einer Subtraktionsstufe oder gegenphasig einer Additionsstufe ADD zugeführt.

Wegen des Amplitudenunterschieds beider Signale ist in die Leitung von der Signalquelle zur Subtraktions- bzw. Additionsstufe ADD ein Regelglied RG eingefügt. Das Regelglied RG enthält einen Dämpfungsregler zum Ausgleich des Amplitudenunterschiedes und einen Phasenregler zum Ausgleich der Phasenverschiebung, den das von der Meßspule MSP zugeführten Signal aufweist.

Am Ausgang der Subtraktions- bzw. Additionsstufe ADD erscheint beim Meßvorgang nach dem Amplituden- und Phasenabgleich ein Restsignal, das in Verbindung mit dem eingestellten Wert der über das Regelglied RG eingestellten Phasenverschiebung eine Aussage über die Impedanz der mit dem Signal beaufschlagten Körperregion ermöglicht. Der Phasenregler in dem Regelglied RG kann entfallen, wenn entsprechend den Ausführungen zu der Figur 12 die Subtraktions- bzw.

Additionsstufe ADD zugleich mit einem Phasenvergleicher versehen ist, der die erwähnte Phasenverschiebung unmittelbar mißt und zur Anzeige an einer Anzeigevorrichtung OSC bringt oder als Einstellgröße an das Bedienpult PA bzw. den Mikroprozessor MP liefert.

In der Figur 14 ist eine Meßeinrichtung gezeigt, die an den Regelverstärker in Figur 12 anschließt. Der Sendespule SSP ist eine Meßspule MSP zugeordnet, die ihrerseits eine Meßeinrichtung oder eine Anzeigeeinrichtung, wie ein Oscilloscop OSC speist. Zwischen Meßspule MSP und Oscilloscop OSC ist ein Schalter SCH eingefügt, der vom Ausgangssignal des Regelverstärkers RV gespeist wird. Die Steuerung des Schalters SCH ist dabei so, daß der Übertragungsweg von der Meßspule MSP zur Anzeigevorrichtung OSC so lange unterbrochen ist, als ein Strom vom Regelverstärker RV an die Sendespule SSP abgegeben wird, d.h., solange Impulse abgegeben werden. In den Impulspausen, also wenn RV in SSW keinen Strom einspeist, wird der Übertragungsweg jeweils geschlossen. Das in den Impulspausen von der Meßspule MSP aufgenommene Signal gelangt damit in der beim Beispiel als Oscilloscop OSC ausgebildeten Anzeigevorrichtung zur Wiedergabe.

Für einen gleichartigen Zweck ist auch die in Figur 13 gezeigte Schaltung einsetzbar, wenn an die Stelle des 100 kHz-Generators SG der Regelverstärker RV nach den Figuren 12 bzw. 14 tritt bzw. angeschaltet ist.

Die mit Schaltungen nach den Figuren 13 und 14 erzielten Meßsignale können in zweierlei Weise für die Regelung der Sendeströme Verwendung finden. Entweder werden die Meßsignale zur Anzeige gebracht und der Bedienende betätigt entsprechend die Einsteller des Bedienpults. Die andere Möglichkeit ist, daß die Meßsignale als unmittelbare Einstellsignale für den Regelverstärker RV bzw. den Mikroprozessor MP verwendet werden, indem sie eine entsprechende Verstärkungsänderung und/oder eine Pulsformänderung veranlassen und/oder die Zeitsteuerung entsprechend ändern.

## Patentansprüche

1. Vorrichtung mit einem niederfrequent gepulsten elektrische Ströme erzeugenden Generator und einer daran angeschlossenen Sendeantenne, deren elektromagnetische Felder zur Beaufschlagung einer zu behandelnden Körperregion dienen,
gekennzeichnet durch ihre Ausbildung zum Transport von Ionen, insbesondere Protonen aus intrakorporalen Elektrolyt-Flüssigkeiten, in und durch die sie umgebenden Gefäßwände und Membranen in der Weise, daß die Sendeenergie so hoch gewählt ist, daß die in die Elektrolyt-Flüssigkeit induzierte Energie höher ist als die thermische Energie und innerhalb der Grenzwerte des sogenannten zellspezifischen Amplitudenfensters liegt, und daß die vom Generator in der, vorzugsweise induktionsarm ausgebildeten Sendespule erzeugten gepulsten Ströme (I) folgende Eigenschaften besitzen:
- der Grundstrompuls (P_{I1}) besteht aus der Überlagerung eines Rechteck-Stromes und eines etwa nach einer e-Funktion ansteigenden Stromes, gefolgt von einer wenigstens gleichlangen Pulspause (P_{P1}),
- die Grundfrequenz der Grundstrompulse (P_{I1}) mit Grundpulspausen (P_{P1}) beträgt 100 bis 1000 Hz, vorzugsweise 200 Hz,
- die Amplitude der Grundpulsfolge (P_{I2}) ist mit einer Modulationsfrequenz von 0,5 bis 35 Hz, vorzugsweise 20 Hz, in der Amplitude moduliert,
- die modulierte Grundpulsfolge (P_{I2}) wird als Pulsfolgeserie (P_{I3}) für eine Zeitdauer von 0,3 bis 1,0 sec. ausgesendet, woran sich jeweils eine Pulsserienpause (P_{P3}) von 0,7 bis 5,0 sec. Anschließt.

2. Vorrichtung nach Anspruch 1,
**dadurch gekennzeichnet,**
daß das Puls-Pausen-Verhältnis der Grundpulse (P_{I1}, P_{P1}) etwa 2:3 beträgt.

3. Vorrichtung nach Anspruch 1 oder 2,
**dadurch gekennzeichnet,**
daß jedem Grundpuls (P_{I1}) Hochfrequenzpulse mit einer Frequenz von 10 bis 100 kHz überlagert sind.

4. Vorrichtung nach Anspruch 1, 2 oder 3,
**dadurch gekennzeichnet,**
daß die Modulationsamplitude der Grundpulsfolge (P_{I2}) etwa ein gleichschenkliges Dreieck bildet.

5. Vorrichtung nach einem oder mehreren der Ansprüche 1 bis 4,
**dadurch gekennzeichnet,**
daß die Grundpulsfolge (P_{I2}) ohne Polaritätswechsel moduliert ist.

6. Vorrichtung nach einem oder mehreren der Ansprüche 1 bis 5,
**dadurch gekennzeichnet,**
daß in der Pulsserienpause (P_{P3}) an die Sendespule (10 ... 70) ein etwa sinusförmiger Meßstrom mit einer Frequenz im Bereich von 100 kHz angelegt wird, und daß eine Empfangsspule (15, 42, 43,44) vorgesehen ist, deren Empfangssignal der Bestimmung der elektrischen Impedanz und der elektrischen Polarisierung der beaufschlagten Körperregion dient.

7. Vorrichtung nach einem oder mehreren der Ansprüche 1 bis 7,
**dadurch gekennzeichnet,**
daß dem, vorzugsweise bezüglich Frequenzen, Amplituden, Kurvenformen bzw. Ein-Aus-Zeiten abstimmbaren Generator eine Biofeedback-Regelung zur Einstellung der optimalen Feldparameter zugeordnet ist.

8. Vorrichtung nach Anspruch 7,
**dadurch gekennzeichnet,**
daß zur Regelgrößengewinnung ein Blutdruckmesser vorgesehen ist.

9. Vorrichtung nach Anspruch 7,
**dadurch gekennzeichnet,**
daß zur Regelgrößengewinnung ein Thermograph vorgesehen ist.

10. Vorrichtung nach Anspruch 7,
**dadurch gekennzeichnet,**
daß zur Regelgrößengewinnung ein Pulsmeßgerät vorgesehen ist.

11. Vorrichtung nach Anspruch 7,
**dadurch gekennzeichnet,**
daß zur Regelgrößengewinnung ein Atemvolumen-Meßgerät vorgesehen ist.

12. Vorrichtung nach Anspruch 7,
**dadurch gekennzeichnet,**
daß zur Regelgrößengewinnung eine Meßspule für die Aufnahme des am Bestrahlungsobjekt reflektierten Magnetfeldes vorgesehen ist.

13. Vorrichtung nach Anspruch 12,
**dadurch gekennzeichnet,**
daß an die Meßspule eine Auswerteschaltung angeschaltet ist, deren Meßsignal über einen Regler die Parameter der Sendestrompulse optimiert.

14. Vorrichtung nach Anspruch 12 oder 13,
**dadurch gekennzeichnet,**
daß die Meßspule kreisförmig ist und nur eine Windung und einen Durchmesser von 20 cm hat, und daß die Sendeenergie so hoch gewählt ist, daß vom reflektierten Magnetfeld in ihr eine Meßspannung von 20 bis 30 mV induziert wird.

15. Vorrichtung nach einem oder mehreren der Ansprüche 7 bis 14,
**dadurch gekennzeichnet,**
daß drei umschaltbare Meßspulen (42, 43, 44) mit unterschiedlichen, an die Abmessungen der zu behandelnden Körperregion angepaßten Durchmessern (d₁, d₂, d₃) vorgesehen sind.

16. Vorrichtung nach einem oder mehreren der Ansprüche 1 bis 15,
**dadurch gekennzeichnet,**
daß die Sendespule derart ausgebildet ist, daß örtliche Feldspitzen des in den Organismus abgestrahlten Feldes vermieden sind.

17. Vorrichtung nach Anspruch 16,
**dadurch gekennzeichnet,**
daß die Windungen der Sendespule einen Quadrupol bilden.

## Claims

1. A device with a generator creating low-frequency pulsed electrical currents and a transmission antenna connected to it, with the electromagnetic fields from the antenna being applied to a region of the body requiring treatment,
characterized in that it is configured for the transport of ions, in particular protons, from intracorporeal electrolytic fluids into and through the vessel walls and membranes which surround them, with the transport being performed in such a manner that the transmission energy selected is of a high enough level so that the energy induced in the electrolytic fluid is greater than the thermal energy and is within the limiting values of the so-called cell-specific amplitude window, and that the pulsed currents (I) created by the generator in the transmission coil, which is preferably a low-induction design, have the following properties:
- the basic current pulse (P_{I1}) consists of a square wave current superimposed on a current rising approximately like an e-function, followed by an interpulse period (P_{P1}) of at least the same duration,
- the basic frequency of the basic current pulses (P_{I1}) with basic interpulse periods (P_{P1}) is 100 to 1000 Hz, preferably 200 Hz,
- the amplitude of the basic pulse sequence (P_{I2}) is modulated with a modulation frequency of 0.5 to 35 Hz, preferably 20 Hz,
- the modulated basic pulse sequence (P_{I2}) is transmitted as a series of pulse sequences (P_{I3}) for a duration of 0.3 to 1.0 seconds and each series is then followed by a interpulse sequence period (P_{P3}) of 0.7 to 5.0 seconds.

2. The device in accordance with Claim 1,
**characterized in that**,
the mark-to-space ratio of the basic pulses (P_{I1}, P_{P1}) is approximately 2:3.

3. The device in accordance with Claim 1 or Claim 2,
**characterized in that**,
High frequency pulses with a frequency of 10 to 100 kHz are superimposed on each basic pulse (P_{I1}).

4. The device in accordance with Claims 1, 2 or 3,
**characterized in that**,
the modulation amplitude of the basic pulse sequence (P_{I2}) approximately forms an isosceles triangle.

5. The device in accordance with one or more of Claims 1 to 4,
**characterized in that**,
the basic pulse sequence (P_{I2}) is modulated without a change in polarity.

6. The device in accordance with one or more of Claims 1 to 5,
**characterized in that**,
an approximately sinusoidal measuring current with a frequency in the region of 100 kHz is applied to the transmitter coil (10 ... 70) in the interpulse sequence period (P_{I3}), and that a receiver coil (15, 42, 43, 44) is provided, the reception signal of which is used for determining the electrical impedance and electrical polarization of the region of the body to which the pulses are applied.

7. The device in accordance with one or more of Claims 1 to 6,
**characterized in that**,
a closed-loop bio-feedback controller for setting the optimum field parameters is allocated to the generator which can preferably be adapted to different frequencies, amplitudes, curve shapes and/or on-off times.

8. The device in accordance with Claim 7,
**characterized in that**,
a blood pressure gauge is provided in order to obtain control parameters.

9. The device in accordance with Claim 7,
**characterized in that**,
a thermograph is provided in order to obtain control parameters.

10. The device in accordance with Claim 7,
**characterized in that**,
a pulse measuring device is provided in order to obtain control parameters.

11. The device in accordance with Claim 7,
**characterized in that**,
a breathing volume measuring device is provided in order to obtain control parameters.

12. The device in accordance with Claim 7,
**characterized in that**,
a measuring coil for recording the magnetic field reflected from the object to which the pulses are being applied is provided in order to obtain control parameters.

13. The device in accordance with Claim 12,
**characterized in that**,
an evaluation circuit is connected to the measuring coil, and the measurement signal from this circuit optimizes the parameters of the transmitted current pulses by means of a controller.

14. The device in accordance with Claims 12 or Claim 13,
**characterized in that,**
the measuring coil is circular in shape and has only one loop with a diameter of 20 cm, and that the energy level of the transmission is selected high enough for the reflected magnetic field to induce a measurement voltage of 20 to 30 mV in the coil.

15. The device in accordance with one or more of Claims 7 to 14,
**characterized in that**,
three interchangeable measuring coils (42, 43, 44) are provided which have diameters (d₁, d₂, d₃) adapted to the dimensions of the region of the body requiring treatment.

16. The device in accordance with one or more of Claims 1 to 15,
**characterized in that**,
the transmitter coil is formed in such a way as to preclude localized field peaks in the field which is radiated in the organism.

17. The device in accordance with Claim 16,
**characterized in that**,
the loops of the transmitter coil form a quadrupole.

## Revendications

1. Dispositif comprenant une génératrice produisant des courants électriques pulsés avec basse fréquence et une antenne émettrice raccordée dont les champs électromagnétiques agissent sur des régions du corps à traiter,
caractérisé en ce qu'il est conçu pour transporter des ions, en particulier des protons provenant de liquides électrolytiques intracorporels, dans et à travers les parois du récipient et membranes l'entourant, de sorte que l'intensité réglée de l'énergie émettrice a pour effet que l'énergie induite dans le liquide électrolytique est plus élevée que l'énergie thermique et se situe dans les limites de ce qu'on appelle la fenêtre d'amplitudes spécifique des cellules, et que les courants pulsés (I) produits par la génératrice dans la bobine émettrice, conçue de préférence à induction réduite, revêtent les caractéristiques suivantes:
- L'impulsion du courant de base (P_{I1}) consiste d'une superposition d'un courant rectangulaire et d'un courant augmentant à peu près selon une équation e, suivis d'une pause d'impulsion (P_{P1}) d'au moins la même durée,
- La fréquence de base des impulsions du courant de base (P_{I1}) avec pauses d'impulsion de base (P_{P1}) s'élève à 100-1000 Hz, de préférence 200 Hz,
- L'amplitude de la fréquence de base des impulsions (P_{I2}) a une fréquence de modulation dans l'amplitude de 0,5 à 35 Hz, de préférence 20 Hz,
- La séquence modulée des impulsions de base (P_{I2}) est émise pendant une durée de 0,3 à 1,0 sec en tant que série d'impulsions (P_{I3}), suivie chaque fois d une pause d'impulsion (P_{P3}) d'une durée de 0,7 à 5,0 sec.

2. Dispositif d'après la revendication 1,
caractérisé en ce que
le rapport impulsion/pause des impulsions de base (P_{I1}, P_{P1}) est d'env. 2:3.

3. Dispositif d'après les revendications 1 ou 2,
caractérisé en ce que
à chaque impulsion de base (P_{I1)}, il est superposé des impulsions haute fréquence d'une fréquence de 10 à 100 kHz.

4. Dispositif d'après les revendications 1, 2 ou 3,
caractérisé en ce que
l'amplitude de modulation de la séquence d'impulsions de base (P_{I2}) forme à peu près un triangle isocèle.

5. Dispositif d'après une ou plusieurs des revendications 1 à 4,
caractérisé en ce que
la séquence d'impulsions de base (P_{I2}) est modulée sans inversion de polarité.

6. Dispositif d'après une ou plusieurs des revendications 1 à 5,
caractérisé en ce que
dans la pause entre les séries d'impulsion (P_{P3}), il est appliqué à la bobine émettrice (10 ... 70) un courant de mesure à peu près sinusoïdal dont la plage de fréquence (15, 42, 43, 44) se situe à 100 kHz, et qu'il est prévu une bobine réceptrice dont le signal récepteur est destiné a la détermination de l'impédance électrique et de la polarisation électrique de la région du corps à traiter.

7. Dispositif d'après une ou plusieurs des revendications 1 à 6,
caractérisé en ce que,
à la génératrice réglable de préférence pour ce qui est les fréquences, les amplitudes, les formes de courbes ou les temps d'activation et de désactivation, il est assigné une régulation à bio-rétroaction pour la régulation des paramètres de champ optimaux.

8. Dispositif d'après la revendication 7,
caractérisé en ce que
pour la détermination du paramètre de régulation, il est prévu un sphygmomanomètre.

9. Dispositif d'après la revendication 7,
caractérisé en ce que
pour la détermination du paramètre de régulation, il est prévu un thermographe.

10. Dispositif d'après la revendication 7,
caractérisé en ce que
pour la détermination du paramètre de régulation, il est prévu un spygmographe.

11. Dispositif d'après la revendication 7,
caractérisé en ce que
pour la détermination du paramètre de régulation, il est prévu un appareil de mesure du volume d'aspiration.

12. Dispositif d'après la revendication 7,
caractérisé en ce que
pour la détermination du paramètre de régulation, il est prévu une bobine de mesure pour recevoir le champ magnétique réfléchi sur l'objet à irradier.

13. Dispositif d'après la revendication 12,
caractérisé en ce que,
à la bobine de mesure, il est raccordé une connexion d'évaluation dont le signal de mesure optimise, par l'intermédiaire d'un régulateur, les paramètres des impulsions du courant d'émission.

14. Dispositif d'après les revendications 12 ou 13,
caractérisé en ce que
la bobine de mesure a une forme circulaire et ne comprend qu'un spire avec un diamètre de 20 cm et que l'intensité de l'énergie d'émission est choisie de sorte à ce que le champ magnétique réfléchi lui induit une tension de mesure de 20 à 30 mV.

15. Dispositif d'après une ou plusieurs des revendications 7 à 14,
caractérisé en ce
qu'il est prévu trois bobines de mesure commutables (42, 43, 44) dont les diamètres différents (d₁, d₂, d₃) sont adaptés aux dimensions des régions du corps à traiter.

16. Dispositif d'après une ou plusieurs des revendications 1 à 15,
caractérisé en ce que
la bobine émettrice est formée de façon à ce que des crêtes de champ locales du champ irradié dans l'organisme soient évitées.

17. Dispositif d'après la revendication 16,
caractérisé en ce que
les spires de la bobine émettrice forment un quadripôle.
